Europäisches Patentamt

European Patent Office

Office européen des brevets

(19)

(11) Publication number: **0 264 478**
**B1**

(12)

# EUROPEAN PATENT SPECIFICATION

(45) Date of publication of the patent specification:
29.08.90

(51) Int. Cl.⁵: **A61B 5/113**

(21) Application number: **86114679.3**

(22) Date of filing: **22.10.86**

(54) **Device detecting breathing.**

(43) Date of publication of application:
**27.04.88 Bulletin 88/17**

(45) Publication of the grant of the patent:
**29.08.90 Bulletin 90/35**

(84) Designated Contracting States:
**DE FR GB IT**

(56) References cited:
**EP-A- 0 019 321**
**US-A- 2 325 688**
**US-A- 3 946 422**
**US-A- 4 175 263**
**US-A- 4 424 419**
**US-A- 4 516 428**

(73) Proprietor: FUKUDA DENSHI CO., LTD., 39-4,
Hongo 3-chome Bunkyo-ku, Tokyo 113(JP)

(72) Inventor: Muraki, Yosiya, 852, Shimoururudo, Hasuda-shi
Saitama-ken(JP)
Inventor: Takahashi, Akinori, 5-16-36, Mihara, Asaka-shi
Saitama-ken(JP)

(74) Representative: TER MEER - MÜLLER - STEINMEISTER
& PARTNER, Mauerkircherstrasse 45,
D-8000 München 80(DE)

ACTORUM AG

## Description

The invention relates to a breathing detection device comprising a sponge body capable of expansion and contracting in response to contractive and expansive motions of a pectoral or abdominal part of a man, a cover enclosing said sponge body from escaping therefrom, a flange provided integrally with the edge of said sponge cover, an air guide tube for guiding an air stream through the cover and from the sponge body and by expansion and contracting of said sponge body and a detector connected to the air guide tube.

A breathing detection device of this type is disclosed by the EP-A 0 019 321.

Heretofore, electrocardiographs have been effectively utilized to determine the health condition of patients. It is necessary to have knowledge of the condition of the breathing organ, i.e., lungs, of a patient who has a breathing disorder. The condition of the breathing organ can be determined by grasping the status of breathing, e.g., the number of breathings per unit time, waveform of breathing, etc.

The pectoral or abdominal part of a man is moved as he or she breathes. This means that the pectoral or abdominal part represents the status of breathing. Therefore, if the movement of the pectoral or abdominal part can be grasped by some or other means, the condition of the breathing organ, i.e., the lungs, can be determined by detecting and recording the status of breathing.

This is very effective as a blooding-free method of monitoring the breathing at the time of an actual surgical operation so long as there is no need of having information of the inside of the lungs.

From this viewpoint, the monitoring of the status of breathing of a patient has heretofore been performed by using an impedance system or a nasal foramen thermistor system. In the former system, the status of breathing is determined through measurement of the impedance of the surface of the patient's body. The impedance noted above is subject to changes with the breathing motion. The status of breathing is thus determined from the impedance changes.

In the latter system, a small thermometer is fitted in the nasal forames. The status of breathing is determined through measurement of ambient temperature changes which are brought about as the patient breathes through the nose.

The status of breathing of a patient can be detected by the above methods. However, with the impedance system it is difficult to detect the status of breathing accurately in case where the sensitivity to the breathing is low or when the breathing is not so deep. Further, the nasal foramen thermistor system often makes use of an electrocardiogram electrode.

In this case, the position of installation of the electrode is limited.

Further, although satisfactory breathing detection sensitivity can be obtained with the nasal foramen thermistor system, it is necessary in this case to fit a thermistor (i.e., thermometer) in the nose. Doing so spoils the appearance of the patient. In addition, pain is felt when the thermistor is held fitted for long time.

This invention has been intended in the light of the above problems, and its object is to provide a breathing detection device, which can readily detect breathing with high sensitivity.

To attain the above object of the invention, the breathing detection device according to the invention is characterized by said detector including an electrete capacitor microphone having an oscillating film for causing a change in the electrostatic capacitance, between said film and a back electrode in response to a movement of air caused due to expansion and contraction of said air bag, and having a field-effect transistor for converting a change in the electrostatic capacitance into a change in voltage, and a pressure reduction film provided in front of said oscillating film for preventing mechanical saturation of said oscillating film when the extent of said movement of air exceeds the maximum deformation of said oscillating film; said detector detecting a status of breathing through electric conversion of a movement of air into an electric signal, said movement of air being caused with expansion and contraction of said air bag caused with breathing.

Fig. 1 is a plan view showing an air bag in an embodiment of the invention;

Fig. 2 is a perspective view showing an essential part of the air bag shown in Fig. 1;

Figs. 3 and 4 are sectional views showing the air bag shown in Fig. 2;

Figs. 5 and 6 are sectional views showing an essential part of a breathing detection device comprising an air bag and a detector;

Fig. 7 is a sectional view showing a detector;

Fig. 8 is an exploded perspective view showing the air bag; and

Figs. 9 and 10 are views for explaining the air bag applied to a pectoral part of a patient in close contact therewith.

Now, the constitution and functions of the invention will be described in conjunction with an embodiment thereof consisting of an air bag 1 and a detector 7 with reference to the drawings.

Further, the description will be made with respect to the air bag 1, which is closely applied to the pectoral or abdominal part of a patient, and in which air is moved with its expansion and contraction in response to the contraction and expansion of the pectoral or abdominal part.

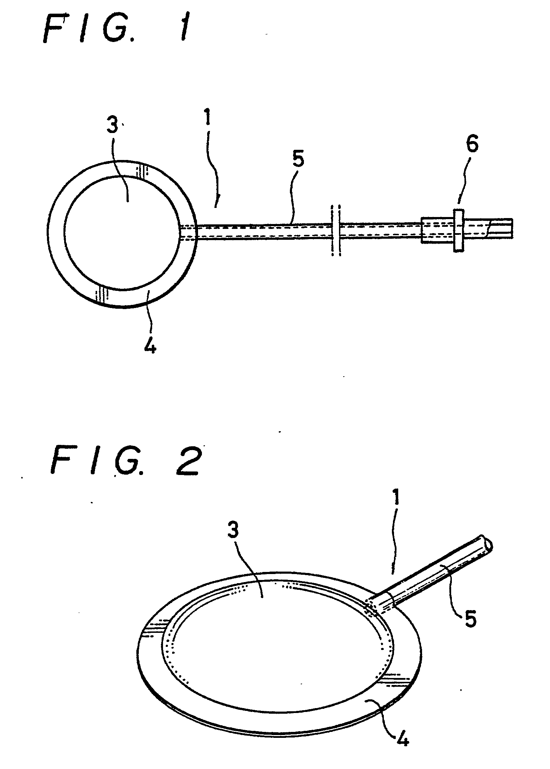

Fig. 1 is a plan view showing an embodiment of the breathing detection air bag according to the invention, Fig. 2 is a perspective view showing an essential part of the air bag shown in Fig. 1, and Figs. 3 and 4 are sectional views of the air bag. Referring to the Figures, there is shown an air bag generally designated at 1. The air bag 1 comprises a sponge body 2, a sponge cover 3 of vinyl chloride, a flange 4 and an air guide tube 5.

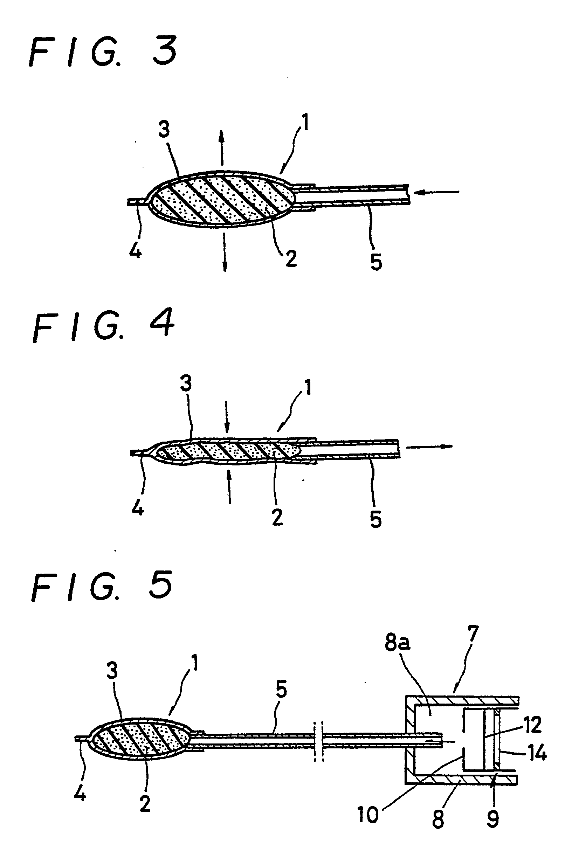

The construction of the air bag will now be described in further detail. The sponge body 2 of the air bag 1 consists of polyurethane synthetic resin foam, which is soft and flexible and has numerous continuous pores. The vinyl chloride sponge cover 3 entirely encloses the sponge body 2 to prevent escapement of air contained in the continuous pores. The sponge body 2 enclosed in the sponge cover 3 is closely applied to the pectoral or abdominal part of the patient. In this state, it is expanded and contracted in correspondence to the movement of the pectoral or abdominal part of the patient caused with the breathing thereby. With the expansion or contraction of the sponge body 2, air is withdrawn into or forced out of the pores of the sponge body 2 enclosed in the sponge cover 3 through the air guide tube 5, as shown in Figs. 3 and 4.

The flange 4 projects outwardly from the edge of the sponge cover 3 enclosing the sponge body 2. The air guide tube 5 extends from one end of the sponge body 2, and a connector 6 is mounted on the other end of the air guide tube 5. The flange 4 is provided in that the air bag 1 can be conveniently separated from the pectoral or abdominal part by pinching it after the air bag has been closely applied to the pectoral or abdominal part using adhesive tape.

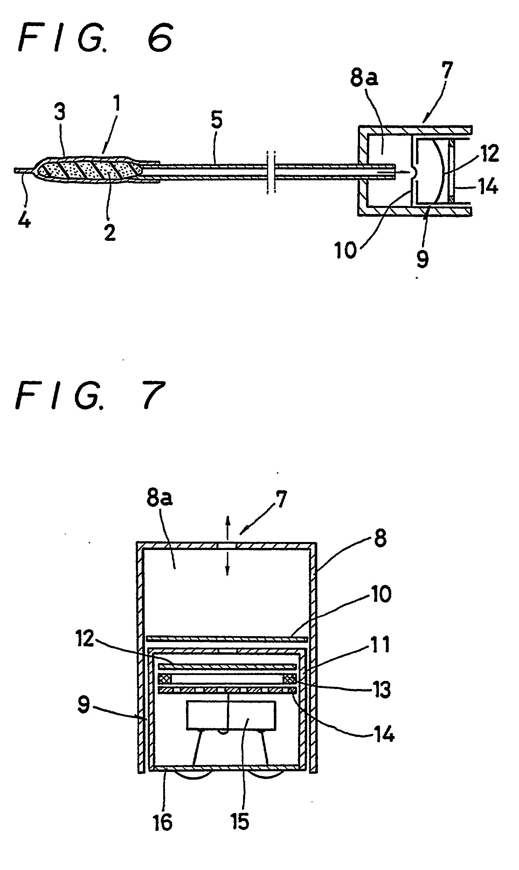

The connector 6 provided at the other end of the air guide tube 5 permits connection of the air guide tube 5 to a detector 7, as shown in Figs. 5 and 6, which detects a movement of air due to expansion or contraction of the sponge body 2 and converts this movement of air into an electric signal. The movement (i.e., distortion) of the oscillating film 12 causes a change in the electrostatic capacitance between the oscillating film 12 and back electrode 14. The field-effect transistor 15 converts this change in the electrostatic capacitance into a change in voltage, whi ch is displayed as the number of breathings per unit time or the waveform of breathing on a display (not shown), thus permitting the detection of the status of breathing.

This breathing detection device is used not only for the detection of the breathing, but it may also be effectively utilized for the detection of the cervical arterial wave in close contact with the top of the cervical arterial skin and for the detection of the finger tip sphygmic wave, apex cordis pulsations, etc.

As has been described in the foregoing, according to the invention the status of breathing is detected by using an air bag. Therefore, there is no need of passing electricity to the skin surface of the living body, that is, electric insulation of the patient is ensured, which is desired form the standpoint of the safety.

Further, the air stream produced with the expansion and contraction of the air bag is detected with a detector. Therefore, satisfactory detection sensitivity can be obtained, and it is possible to ensure accurate detection of the breathing status.

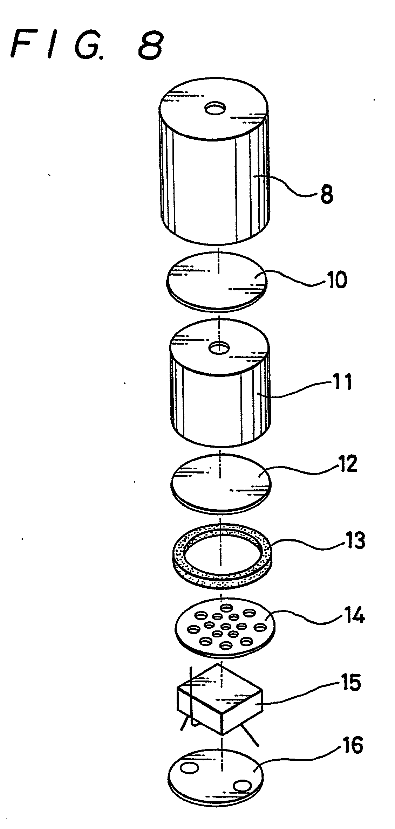

Now, the construction of the detector 7 for converting a movement of air into an electric signal will be described with reference to Figs. 7 and 8. Referring to the Figures, reference numeral 8 designates an outer cylinder of the detector 7. An electrette capacitor microphone 9 is fitted in a lower portion of the detector outer cylinder 8. An air chamber 8a is defined between the outer cylinder 8 and electrette capacitor microphone.

The electret capacitor microphone 9 comprises an electret capacitor microphone outer cylinder 11, which accommodates an oscillating film 12, a spacer 13, a back electrode 14 and a field-effect transistor 15 and has its lower open end closed by a base member 16. A pressure reduction film 10 is located over the electrette capacitor microphone outer cylinder 11.

The oscillating film 12 is deformed in response to the extent of movement of air which is introduced through the air guide tube 5 into the air chamber 8a with the expansion and contraction of the air bag 1. Thus, a change in the electrostatic capacitance between the oscillating film 12 and back electrode 14 is produced. This change in the electrostatic capacitance is converted by the field-effect transistor 15 into a change in voltage, which is displayed as the number of breathings per unit time or as the waveform of breathing by a display device (not shown). The maximum deformation extent of the oscillating film 12 of the electrette capacitor microphone 9 is determined depending on its shape. Therefore, if the extent of movement of air exceeds the maximum deformation level, the oscillating film 12 is mechanically saturated to result in saturation of the output voltage.

To avoid this, it is made possible to readily adjust the sensitivity by applying a pressure reduction film to the front side of the oscillating film 12 or by varying the thickness or material of the pressure reduction film 10.

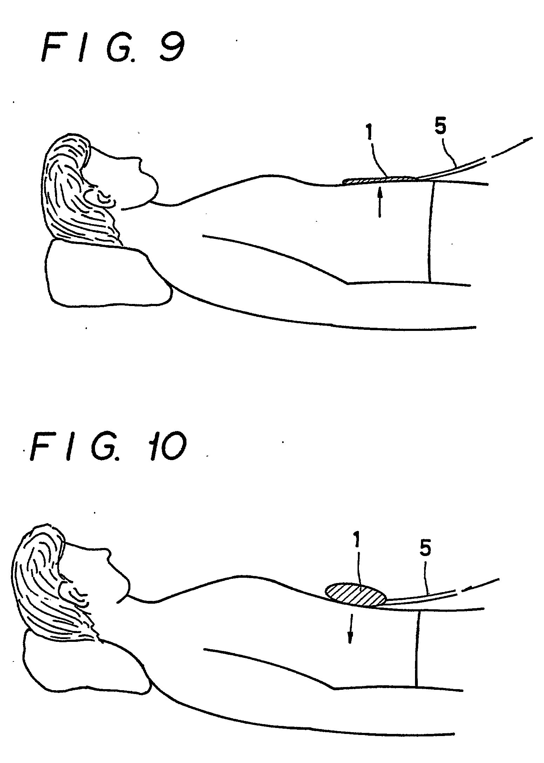

Now, a method of use of the breathing detection device comprising the air bag 1 and detector 7 will be described. As shown in Figs. 9 and 10, the air bag is applied to the abdominal part of a patient with adhesive tape. With the expansive and contractive motions of the patient caused with the breathing thereof, the air bag is caused to undergo contraction and expansion. When the abdominal part is expanded, as shown in Fig. 9, the air bag 1 is urged and contracted so that air contained in the air bag 1 is forced out through the air guide tube 5 into the air chamber 8a, as shown in Fig. 6. The air forced into the air chamber 8a urged the oscillating film 12 via the pressure reduction film 10.

When the pectoral part is contracted, as shown in Fig. 10, the air bag 1 is expanded, and air that has been forced into the detector 7 is pulled through the air guide tube 5 to the air bag 1, shown in Fig. 5, and the oscillating film 12 restores the initial position. The movement (i.e., distortion) of the oscillating film 12 produces a change in the electrostatic capacitance between the oscillating film 12 and back electrode 14. The transistor 15 converts the change in the electrostatic capacitance into a change in the voltage. This change in the voltage is displayed as the number of breathings per unit time and the waveform of breathing on a display (not shown). In this state, the status of breathing is detected.

The breathing detection device may be used not only for the breathing detection as described above, but it may also be effectively utilized for the detection of the cervical arterial wave in close contact with the top of the cervical arterial skin and also for the detection of finger tip sphygmic wave, apex cordis pulsations, etc.

As has been described in the foregoing, according to the invention the status of breathing is detected using the air bag. Therefore, there is no need of causing electricity to the skin surface of a man, and safety can be ensured satisfactorily.

Further, since the air stream due to the expansion and contraction of the air bag is detected with the detector, the status of breathing can be detected accurately while ensuring satisfactory detection sensitivity.

## Claims

1. A breathing detection device, comprising a sponge body (2) capable of expansion and contracting in response to contractive and expansive motions of a pectoral or abdominal part of a man a cover (3) enclosing said sponge body for preventing air contained in the pores of said sponge body form escaping therefrom, a flange (4) provided integrally with the edge of said sponge cover, an air guide tube (5) for guiding an air stream from the cover and the sponge body and caused by expansion and contracting of said sponge body; and a detector (7) connected to the air guide tube characterized by said detector (7) including an electret capacitor microphone (9) having an oscillating film (12) for causing a change in the elctrostatic capacitance between said film and a back electrode (14) in response to a movement of air caused due to expansion and contraction of said air bag, and having a field-effect transistor (15) for converting the change in the electrostatic capacitance into a change in voltage, and a pressure reduction film (10) provided between said oscillating film and the air guide tube preventing mechanical saturation of said oscillating film when the extent of said movement of air exceeds the maximum deformation of said oscillating film.

2. A breathing detection device as claimed in claim 1, characterized in that the capacitor microphone (9) comprises an outer cylinder (11) accomodating the oscillating film (12), a spacer (13) separating oscillating film and back electrode, the back electrode (14) and the field-effect transistor (15).

3. A breathing detection device as claimed in claim 2, characterized in that the pressure reduction film (10) is located over the outer cylinder (11) of the capacitor microphone.

## Patentansprüche

1. Atemabtastvorrichtung, mit einem Luftbeutel (1) mit Schwammkörper (2), der ausdehnbar und zusammenziehbar entsprechend kontraktiven und expansiven Bewegungen eines Brust- oder Bauchbereichs eines Menschen ist, mit einer Schwammabdeckung (3), die den Schwammkörper einschließt und verhindert, daß in den Poren des Schwammkörpers enthaltene Luft aus diesem austritt, einem Flansch (4), der einstückig mit dem Rand der Schwammabdeckung ausgebildet ist, und einem Lufteinlaßrohr (5) zur Führung eines Luftstroms, der erzeugt wird durch Ausdehnung oder Zusammenziehung des Schwammkörpers, und einem Detektor (7), dadurch gekennzeichnet, daß der Detektor (7) ein Elektret-Kondensator-Mikrophon (9) umfaßt, das einen hin- und hergehenden Film (12) aufweist, der eine Änderung der elektrostatischen Kapazität zwischen dem Film und einer Gegenelektrode (14) entsprechend einer Luftbewegung aufgrund einer Ausdehnung oder Zusammenziehung des Luftbeutels erzeugt, und einen Feldeffekttransistor (15) aufweist, der eine Änderung der elektrostatischen Kapazität in eine Spannungsänderung umwandelt, und einen druckreduzierenden Film (10), der vor dem oszillierenden Film angeordnet ist und eine mechanische Sättigung des oszillierenden Filmes verhindert, wenn das Ausmaß der Bewegung der Luft die Verformung des oszillierenden Films des Detektors überschreitet, der den Status der Atmung durch elektrische Umwandlung einer Bewegung der Luft in ein elektrisches Signal abtastet, welche Bewegung der Luft durch Ausdehnung und Zusammenziehung des Luftbeutels aufgrund der Atmung verursacht wird.

2. Atemabtastvorrichtung gemäß Anspruch 1, dadurch gekennzeichnet, daß das Kondensator-Mikrophon (9) einen äußeren Zylinder (11) umfaßt, der den oszillierenden Film (12), ein Distanzstück (13), die Gegenelektrode (14) und den Feldeffekttransistor (15) aufnimmt.

3. Atemabtastvorrichtung gemäß Anspruch 2, dadurch gekennzeichnet, daß der druckreduzierende Film (10) über dem äußeren Zylinder (11) des Kondensator-Mikrophons angeordnet ist.

## Revendications

1. Dispositif de détection de la respiration, comportant un corps spongieux (2) pouvant se dilater et se contracter en réponse à des déplacements de contraction et de dilatation d'une partie pectorale ou abdominale d'un être humain, une enveloppe (3) entourant ledit corps spongieux pour empêcher l'air contenu dans les alvéoles dudit corps spongieux de s'en échapper, un rebord (4) faisant corps avec le bord de ladite enveloppe du corps spongieux, un tube de guidage d'air (5) pour guider un flux d'air depuis l'enveloppe et le corps spongieux et provoqué par la dilatation et la contraction dudit corps spongieux; et un détecteur (7) relié audit tube de guidage d'air, caractérisé en ce que ledit détecteur (7) comprend un microphone condensateur à électret (9) possédant une pellicule oscillante (12) pour provoquer une variation de la capacité électrostatique entre ladite pellicule et une électrode arrière (14) en réponse à un mouvement de l'air provoqué par une dilatation et une contraction dudit corps spongieux, et possédant un transistor à effet de champ (15) pour convertir la variation de capacité électrostatique en une variation de tension, et une pellicule de réduction de pression (10) prévue entre ladite pellicule oscillante et ledit tube de guidage d'air pour empêcher une saturation mécanique de ladite pellicule oscillante lorsque l'étendue dudit mouvement de l'air dépasse la déformation maximale de ladite pellicule oscillante.

2. Dispositif de détection de la respiration selon la revendication 1, caractérisé en ce que le microphone condensateur (9) comporte un cylindre extérieur (11) recevant la pellicule oscillante (12), une pièce d'écartement (13) séparant la pellicule oscillante et l'électrode arrière, l'électrode arrière (14) et le transistor à effet de champ (15).

3. Dispositif de détection de la respiration selon la revendication 2, caractérisé en ce que la pellicule de réduction de pression (10) est située au-dessus du cylindre extérieur (11) du microphone condensateur.

F I G. 1

F I G. 2

# F I G. 3

# F I G. 4

# F I G. 5

# F I G. 6

# F I G. 7

# FIG. 8

# F I G. 9

# F I G. 10